# EUROPEAN PATENT APPLICATION

(11) **EP 3 663 784 A1**
(43) Date of publication of application: **10.06.2020**
(21) Application number: 18211046.0
(22) Date of filing: 07.12.2018
(51) Int. Cl.: G01R 33/34, A61B 5/055, G01R 33/3415, G01R 33/36

(54) **MODULAR MAGNETIC RESONANCE IMAGING SUPPORT**

(71) Applicant: Koninklijke Philips N.V., 5656 AG Eindhoven (NL)
(72) Inventor: HARVEY, Paul Royston, 5656 AE Eindhoven (NL); POSSANZINI, Cecilia, 5656 AE Eindhoven (NL); HAM, Cornelis Leonardus Gerardus, 5656 AE Eindhoven (NL); VAN LIERE, Filips, 5656 AE Eindhoven (NL)
(74) Representative: de Haan, Poul Erik

(57) **Abstract**

The invention provides for a medical instrument (10, 20, 30) comprising a subject support (100). The subject support comprises a table top (108) configured for translating at least a portion of a subject within an imaging zone of a magnetic resonance imaging system (112). The table top comprises: as least one receptacle (304) recessed into the table top; a cable system (308) configured for providing power and/or connectivity to a magnetic resonance imaging coil (412); and multiple connectors (120) distributed within the at least one receptacle. The multiple connectors are configured for providing access to the power and the connectivity of the cable system.

## Description

### TECHNICAL FIELD

The invention relates to magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a subject. This large static magnetic field is referred to as the B0 field or the main magnetic field. Various quantities or properties of the subject can be measured spatially using MRI. Various imaging protocols can be implemented by using pulse sequences to control the acquisition of magnetic resonance data. In the design of these pulse sequences there are typically a large number of adjustable image acquisition parameters. For different magnetic resonance imaging protocols, various types of magnetic resonance imaging antennas may be used to acquire the magnetic resonance imaging data. Often times the magnetic resonance imaging antennas need to be changed or reconfigured between different protocols.

United States patent application publication US 2008/0191696A1 discloses a method of imaging a subject with a patient support with an integrated spine coil. A region of a imaging subject to be imaged is longer along a translation axis than an imaging field of view. The imaging subject and a radio frequency coil are translated together along the translation axis in an inward direction respective to the scanner. The inward translating of the radio frequency coil is stopped at a loaded position. Subsequent to the stopping, the imaging subject is further translated in the inward direction while the radio frequency coil remains stationary so that the region of the subject to be imaged translates through a stationary field of view of the stationary radio frequency coil. During the further translating, the region is imaged using the stationary radio frequency coil and the magnetic resonance imaging scanner.

### SUMMARY OF THE INVENTION

The invention provides for a medical instrument and a modular table insert in the independent claims. Embodiments are given in the dependent claims.

Embodiments may provide for a means of more easily reconfiguring a magnetic resonance imaging system. This may be accomplished by building a cable system into the table top of a subject support. Various modular table inserts are able to connect to this cable system and provide for the modular modification of coils and connections which are built into subject support. The integrating of electronics into modular table inserts may enable a system configuration modularity for an entire product portfolio with a single mechanical design of the patient support. When the cable system provides digital connectivity, it may also enable configuring the number of MRI receive channels provided by a particular system configuration.

In one aspect the invention provides for a medical instrument comprising a subject support. The subject support comprises a table top configured for translating at least a portion of the subject within an imaging zone of a magnetic resonance imaging system. When the table top is connected to a magnetic resonance imaging system the translational axis of the magnetic resonance imaging system for example the translational axis may be along the z-axis of the magnetic resonance imaging system.

The table top comprises at least one receptacle recessed into the table top. The table top further comprises a cable system configured for providing power and/or connectivity to a magnetic resonance imaging coil. The cable system may take different forms in different examples. In one instance it may provide power, for example there may be a galvanic connection providing power. The connectivity may include analogue connectivity such as a transmission line for a send or receive for a magnetic resonance imaging system. The connectivity may also include digital connectivity. The digital connectivity could for example be provided via a fiber optic, via a digital signal transmitted on galvanic connection, via an ethernet cable such as CAT7, and/or may also be provided via a power line. For example, a digital transmission could also be transmitted along a galvanic connection used for providing power.

The table top further comprises multiple connectors distributed within the at least one receptacle. The connectors are configured for providing access to the power and the connectivity of the cable system. This embodiment may be beneficial because it may provide for an efficient means of having a subject support that can be configured with magnetic resonance imaging coils in a variety and modular way.

In another embodiment the at least one receptacle is at least one groove. The at least one groove may for example be parallel or perpendicular to the translational axis. The at least one groove may also be referred to as a channel or a long narrow cut or depression in the subject support

In another embodiment the tabletop is configured for translating the tabletop along a translational axis. The term translating the table top along a translational axis may also be interpreted as translating the table top linearly.

In another embodiment the multiple connectors are distributed parallel or perpendicular to the translational axis within each of the at least one groove.

In another embodiment the cable system provides power and digital connectivity to the at least one magnetic resonance imaging coil. This embodiment may be advantageous because it may provide for a means of scaling the use of different magnetic resonance imaging channels without having to provide additional radio-frequency architecture. The digitizer and other electronic components can be provided by the magnetic resonance imaging coils themselves. By connecting to the digital connectivity of the cable system the number of channels can therefore be scaled.

The digital connectivity may be provided in different means. In one embodiment the digital connectivity is provided by an optical fiber. The multiple connectors may then have optical connectors which enable this connectivity. In other embodiments the power may be provided by a galvanic connection. The galvanic connection may have a digital signal superimposed upon it so that the single power cable can be used for providing both power and digital connectivity to a magnetic resonance imaging coil.

In another embodiment the digital connectivity is configured for providing the addition of magnetic resonance imaging channels to the medical instrument on the fly. For example, magnetic resonance imaging coils may have digitizers and control electronics built into them. The additional channels can then simply be added to the magnetic resonance imaging system or medical instrument by plugging them into or connecting them to the cable system. This may provide for an inexpensive means of customizing the medical instrument. The term on the fly may be interpreted as being equivalent to the terms plug and play or self configuring. In another embodiment the cable system is a cable system and the connectivity is digital connectivity. This may provide a digital interface to magnetic resonance imaging coils. This may be beneficial because it may simplify the connectors and readily provides MRI receive channel scalability.

In another embodiment the receptacle has a base or lowest point. The connectors may be located at the base of the groove. This may have the advantage of moving the connector as far away from the subject as possible. In other embodiments the connector is elevated above or away from the lowest point. This may be advantageous, because it minimizes the chance that spilled fluids will enter the connector.

In another embodiment the medical instrument further comprises a magnetic resonance imaging system configured for acquiring magnetic resonance imaging data from the imaging zone. The magnetic resonance imaging system is further configured to acquire the magnetic resonance data via the connectivity of the cable system. In the case where the connectivity is analogue the magnetic resonance data may be acquired using a transmitter and/or receiver. In the case where the connectivity is digital the magnetic resonance imaging system is configured to send and receive magnetic resonance imaging coil control commands via the connectivity of the cable system.

In another embodiment the magnetic resonance imaging system comprises a bridge. The table top is configured for fitting within and sliding within the bridge of the magnetic resonance imaging magnet.

In another embodiment the at least one receptacle recessed into the table top is two grooves recessed into the table top. The cable system is positioned within the central support structure of the table top that runs parallel to the translational axis. The central support structure is formed between the two grooves. This embodiment may be beneficial because the two grooves may provide mechanical stability for inserts inserted into the table top. Placing the cable system within the central support structure may be useful in reducing the effect of the cable system on the quality of the magnetic resonance images acquired with the magnetic resonance imaging system.

In another embodiment the at least one groove recessed into the table top is a single groove recessed into the table top. The single groove may be positioned within a central region of the table top. The cable system may be placed below the central groove.

In another embodiment the at least one receptacle is multiple apertures. An aperture as used herein encompasses an opening and a volume within the subject support. One of the multiple connectors is distributed in each of the multiple apertures. The use of apertures may provide for an efficient means of aligning modular magnetic resonance imaging coils or a modular table insert.

In another embodiment the connector is located in the bottom of the aperture. The aperture may have a flap which covers the aperture when there is no modular table insert inserted into that particular aperture. The modular table insert may for example have a connector which pushes aside the flap of the connector in the receptacle.

In another embodiment the medical instrument further comprises at least one modular table insert that has been inserted into the at least one receptacle.

In another embodiment the at least one receptacle is configured for receiving the at least one modular table insert. The at least one modular table insert is configured for forming a support surface constructed for receiving the subject.

In another embodiment the at least one modular table insert is configured for connecting to at least one of the connectors.

In another embodiment the modular table insert comprises a mechanical extension for each of the at least one receptacle in the table top. Each mechanical extension is configured for being inserted into one of the at least one receptacles of the table top.

In another embodiment the modular table insert comprises a table insert connector configured for connecting to one of the multiple connectors when inserted into the table top.

In another embodiment the medical instrument further comprises a mattress that has been placed on top of the table top and covers the at least one modular table insert. This may be beneficial because it may provide for an efficient means of making the modular table inserts more comfortable.

In another embodiment the at least one receptacle is configured for receiving the at least one modular table insert using any one of the following: a gravitationally secured fit, a snap fit, and combinations thereof. In one example the combination of the receptacle and the connector may be enough to secure the modular table insert in place. In other examples there may be an additional mechanism which causes a snap fit.

In another embodiment the at least one receptacle and the multiple connectors are arranged such that each of the at least one modular table inserts can be inserted into the table top with a first angular orientation and a second angular orientation with respect to the translational axis. The first angular orientation and the second angular orientation differ by 180°. This means that the inserts can be inserted in one way or they can be flipped around and also inserted in the other direction. This may be beneficial because it may enable more precise positioning of coil elements within the modular table insert.

In another embodiment each of the at least one receptacle is configured for providing air cooling. For example, each receptacle may have ventilation grills for channeling cooling air provided by the system.. This may be beneficial because it may provide for an effective means of cooling electronics that are part of a modular table insert. For example, electronics can be positioned near the base of the receptacle. The air cooling may then enable the excess heat from the electronics to be taken away before it heats the subject.

In another embodiment the subject support is dockable to the magnetic resonance imaging system. For example, the subject support may be wheeled or otherwise be able to be moved around. The subject support can then dock or mechanically couple to the magnetic resonance imaging system. In some embodiments the mechanical coupler between the magnetic resonance imaging system and the subject support may also provide a connection between the magnetic resonance imaging system and the cable system. This may be beneficial because it may provide for an efficient means of connecting the subject support to the magnetic resonance imaging system.

In another embodiment the table top comprises two opposing end regions. At least one of the two opposing end regions comprises any one of the following: a connection to the cable system, an analogue radio-frequency connector, and combinations thereof. It may be beneficial to have connectors on the opposing end regions because it may enable legacy or old magnetic resonance imaging coils to be connected to the magnetic resonance imaging system. The analogue radio-frequency connector may be used to connect coils for performing transmission and/or receiving during the acquisition of magnetic resonance imaging data.

In another aspect the invention provides for a modular table insert that is configured for being inserted into the table top according to an embodiment. The at least one receptacle of the table top is configured for receiving the at least one modular table insert. The at least one modular table insert is configured for forming a support surface configured for receiving the subject. Each of the at least one modular table insert is configured for connecting to one of the connectors. The modular table insert comprises a mechanical extension for each of the at least one receptacle of the table top. Each mechanical extension is configured for being inserted into one of the at least one receptacles of the table top. The modular table insert comprises a table insert connector configured for connecting to one of the multiple connectors when inserted into the table top.

The modular table insert may be beneficial because it may provide for a means of configuring a subject support in a modular means. The table insert connector may provide power and/or connectivity to a magnetic resonance coil which is embedded or placed within the modular table insert.

In another embodiment the modular table insert comprises a magnetic resonance imaging coil connected to and/or powered by the table insert connector. This may be beneficial because when the table insert is inserted into the table top the modular table insert forms a portion of the table top. The use of the antennas in the modular table insert may then be a means of providing flexible configuration of the subject support.

In another embodiment the magnetic resonance imaging coil is a surface coil.

In another embodiment the magnetic resonance imaging coil is a head coil.

In another embodiment the magnetic resonance imaging coil is a neck coil.

In another embodiment the magnetic resonance imaging coil is a foot coil.

In another embodiment the magnetic resonance imaging coil is a knee coil.

In another embodiment the magnetic resonance imaging coil is a spine coil.

In another embodiment the modular table insert further comprises an analogue magnetic resonance imaging coil connector. In this case the connectivity comprises a transmission line for connecting the magnetic resonance imaging coil to a transmitter and/or a receiver. Digitization of the magnetic resonance imaging data may for example take place within the analog magnetic modular table insert. In some examples additional digital control to control the tune/detune of the magnetic resonance imaging coil may be provided.

In another embodiment the modular table insert further comprises a Wi-Fi connector or wireless connector or protocol system. The wireless connector may for example be useful for connecting to a wireless magnetic resonance imaging coil.

In another embodiment the modular table insert further comprises a digital magnetic resonance imaging coil connector. For example, the connectivity of the cable system may include a means for transmitting and receiving digital information to the magnetic resonance imaging coil.

In another embodiment the modular table insert further comprises an electronic portion. The electronic portion is located in an end region of the mechanical extension. The mechanical extensions are configured for being inserted into a receptacle. By having them at the end region of a mechanical extension the electronics is moved away further from the support surface that supports or receives the subject. This may be beneficial because the electronic portion may generate heat during the operation of the magnetic resonance imaging coil. Taking this heat further away from the subject may improve the comfort of the subject. It may also make it easier to cool the electronics. For example, if air is blown down the groove the air may take the heat away before it has a chance to heat and reach the subject. Another advantage may be that many electronic components have (even traces of) magnetic content (typically iron and/or nickel) that influence the static or dynamic magnetic field (B0) and/or the electro-magnetic RF transmit field (B1). These influences consequently distort the acquired images. Placing electronics further away from the subject reduces (possibly eliminates) such influence.

In another embodiment the modular table insert further comprises a contoured table surface. The modular table inserts may in principle have any shape or configuration on a top region. By having a contoured table surface the modular table insert can be used to replicate the shape or contour of a legacy or prior magnetic resonance imaging subject support. This for example may be useful in attaching specialty or older magnetic resonance imaging coils.

In another embodiment the modular table insert further comprises a mounting structure for a surface mountable magnetic resonance imaging coil. For example, specialized hardware for mounting specialized magnetic resonance imaging coils may be incorporated into a modular table insert. For example, a specialized head coil or other magnetic resonance imaging coil may need a special or particular mounting structure. This can be provided via the modular table insert. This may also have the benefit that when the use of the specialized magnetic resonance imaging coil is finished the modular table insert can simply be removed and replaced with another one that does not have the mounting structure.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig. 1 illustrates an example of a medical imaging system;
Fig. 2 illustrates a further example of a medical imaging system;
Fig. 3 illustrates an example of a subject support;
Fig. 4 illustrates an example of a modular table insert;
Fig. 5 illustrates a further example of a subject support;
Fig. 6 illustrates a further example of a subject support;
Fig. 7 illustrates a further example of a subject support;
Fig. 8 illustrates a further example of a subject support;
Fig. 9 illustrates a further example of a subject support;
Fig. 10 illustrates a further example of a subject support;
Fig. 11 illustrates a further example of a subject support;
Fig. 12 illustrates a further example of a subject support;
Fig. 13 illustrates a further example of a subject support;
Fig. 14 illustrates a further example of a subject support;
Fig. 15 illustrates a further example a modular table insert;
Fig. 16 illustrates a further example of a subject support;
Fig. 17 illustrates a further example of a modular table insert; and
Fig. 18 illustrates a further example of a medical imaging system.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

Examples relate to a subject support and receiving coil platform of an MRI system. Examples may provide for the placement, below the subject, of various configurations of multi-element coils in the form of tiles. In the preferred embodiment, the coil tiles (modular table insert 402) consist of a flat top surface forming a hollow container with protrusions (mechanical extensions 404) extending outward from the bottom. The volume immediately below the top surface is used to contain the coil antenna elements (magnetic resonance imaging coil 412) while the protrusions extending from the bottom are used to contain coil receiving and auxiliary electronics (electronic portion 408), e.g. digital receiver module, in such a manner as to maximize the physical distance between said electronics, the antenna upper surface and consequently the subject. The protrusions (mechanical extensions 404) also provide a convenient location for the coil side electrical, optical and thermal interfaces to the subject carrier and tabletop. The subject support, carrier and tabletop are also formed in a complimentary manner containing multiple recesses, in the form of troughs (grooves 304), which accept a variety of coil tile types at multiple positions/stations along the length of the tabletop. The coil tiles are replaceable and the novel carrier and tabletop design supports multiple configurations providing the mechanical, electrical, optical and thermal interface between the coil tiles and the system.

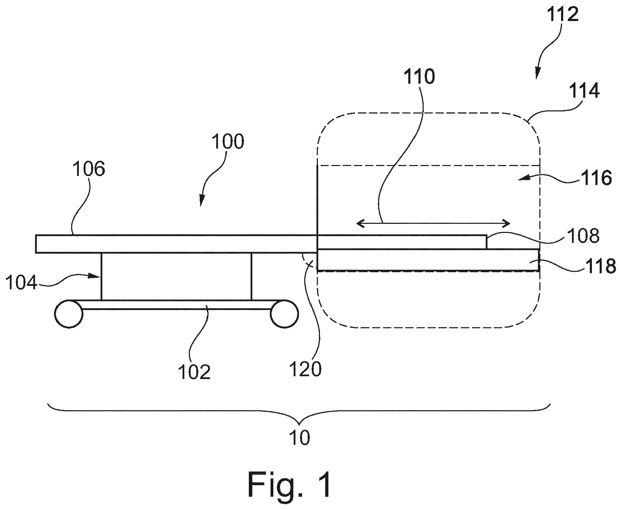

Fig. 1 illustrates an example of a medical instrument 10. The medical instrument 10 comprises a subject support 100 and a magnetic resonance imaging system 112. The subject support 100 is dockable to the magnet 114 of the magnetic resonance imaging system 112. The subject support 100 comprises a transport 102 which in this case is wheels. The subject support 100 also comprises a pedestal 104 which supports a carrier 106. The subject support 100 further comprises a table top 108 which is able to move within the carrier 106. The table top 108 is able to transport a subject into the magnet 114. The table top 108 moves parallel to a translational axis 110. In this example the translational axis 110 is aligned with the z-axis of the magnetic field generated by the magnet 114. The magnetic resonance imaging system 112 comprises a magnet 114. The magnet 114 has a bore 116. A bridge 118 within the bore 116 of the magnet 114 supports the table top 108 as it moves into the magnet 114. When the subject support 100 docks to the magnet 114 there is an optional connector 120. This provides a mechanical connection between the power, radio-frequency and/or digital communication of the magnetic resonance imaging system with the cable system of the subject support 100. The cable system is not depicted in Fig. 1.

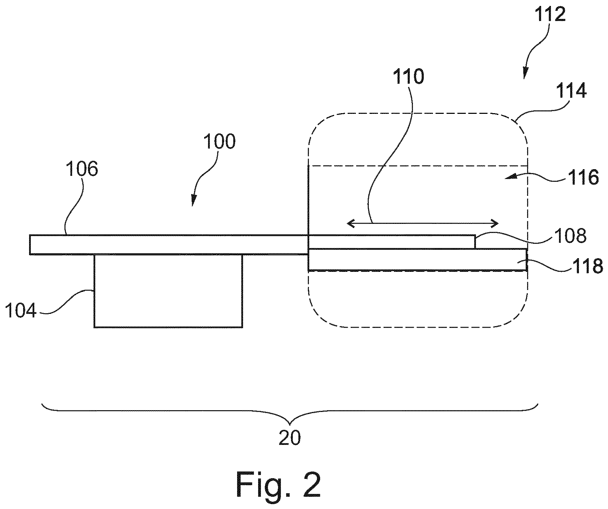

Fig. 2 illustrates a further example of a medical instrument 20. The medical instrument 20 in Fig. 2 is similar to the medical instrument 10 in Fig. 1 with the exception that the subject support 100 is fixed to the magnet 114 and is not dockable. If the docking connector is digital it can be inexpensive and then it may therefore become advantageous to use the docking connector 120 even for a fixed support as this would standardize the magnet assembly and allow (at some later point in time) a fixed configuration to be upgraded to a dockable configuration.

Figs. 1 and 2 may also comprise a computer system for controlling the operating and function of the medical instrument and also for communicating with the connectivity provided by the cable system. The computer may therefore be used to control the magnetic resonance imaging system to acquire magnetic resonance imaging data and to reconstruct the magnetic resonance imaging data into a magnetic resonance image. They may also both comprise an actuator for moving the table top into the bore 116 of the magnetic resonance imaging magnet 114.

Figs. 1 and 2 illustrates the high-level architecture of a subject support/coil platform (subject support 100) that will be referred to in the following descriptions. Two variants are considered here. The subject support and mobile/dockable subject support are illustrated in Fig. 1. The tabletop 108 is considered to be the part of the subject support that physically connects with the coil tiles and carries the tiles (modular table inserts) with subject into and out of the bore of the system. The carrier 106 is part of the subject support that accommodates the tabletop and the mechanics required to move the tabletop in and out of the bore of the system. The bridge piece 118 forms an extension of the carrier that is fixed within the bore of the system and acts primarily as a support and guide rail for the tabletop as it moves in and out.

The pedestal 104 provides the vertical support for the carrier and tabletop and may also include a mechanism to move the carrier/tabletop vertically. For a fixed configuration, there is no transport and the pedestal interfaces directly to the floor. For the mobile configuration, the pedestal 104 interfaces to a transport with wheels. The transport will also contain a means (docking interface or connector for the cable system 120) to provide various electrical, optical and thermal interfaces between the coils, subject support and rest of the system.

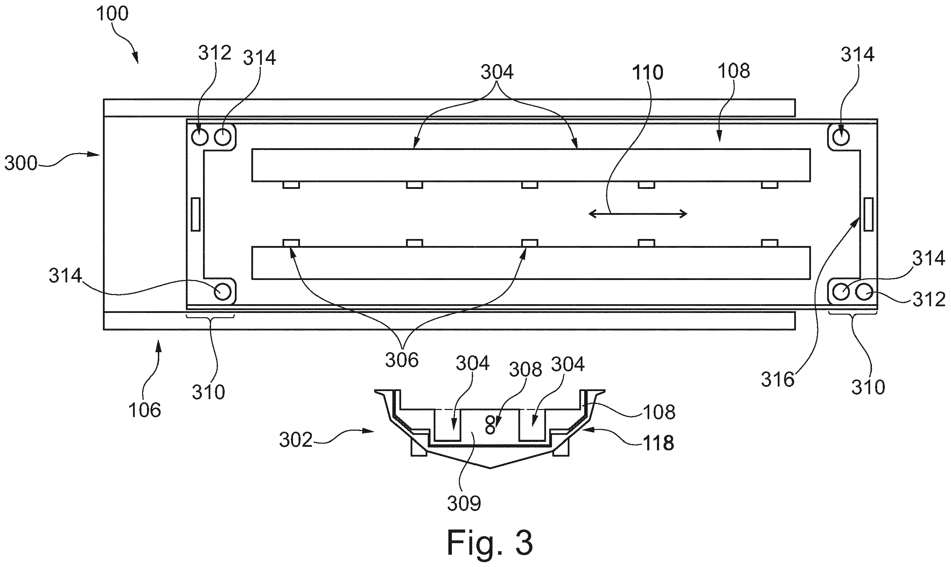

Fig. 3 shows a more detailed view of the subject support 100. The subject support 100 is shown in a top view 300 and a cross-sectional view 302. The table top 108 is shown as having two grooves 304 in it. The two grooves have connectors 306 distributed along them. The connectors 306 are connected to a cable system 308 which may also be referred to as a cable management system. The cable system 308 may provide power, a digital connection, and/or a radio-frequency connection to modular table inserts which are inserted into the table top 108. The cable system 308 in this example is shown as running through the middle of the table top in a central support structure 309. The central support structure 309 is formed by the two grooves 304 on either side. The table top 108 is shown as having opposing end regions 310. In each opposing end region 310 there is an analogue radio-frequency connector 312 which allows an external magnetic resonance antenna to be connected to a transmitter and/or receiver. There is also a connection 314 to the cable system 308. There are also optionally positions 316 for connecting a nurse call or headphone connection 316.

As mentioned above, Fig. 3 illustrates a top view 300 and cross-sectional view 302 of the tabletop 108. As illustrated, the tabletop 108 contains parallel recesses or troughs (grooves 304) running along the length (parallel to the translational axis 110). The grooves 304 could also be placed perpendicular to the translational axis 110.

From the cross-sectional view 302 it is shown that the troughs 304 are positioned either side of the center line and the cable management system (CMS) (cable system 308 runs along the central line. The CMS 308 is the primary power and data bus for every coil tile that connects on the tabletop. The rectangles 306 represent electrical/optical/thermal interface points or connectors 306, within the troughs 304, that extend at regular intervals along the length of the tabletop. We refer to the connection interfaces or connectors 306 within the troughs as Direct Connect DCI connectors (Direct Connect Digital Coil Interface) herein. In such a manner, it is possible to populate the subject tabletop with multiple tiles (modular table inserts) at various locations along its length.

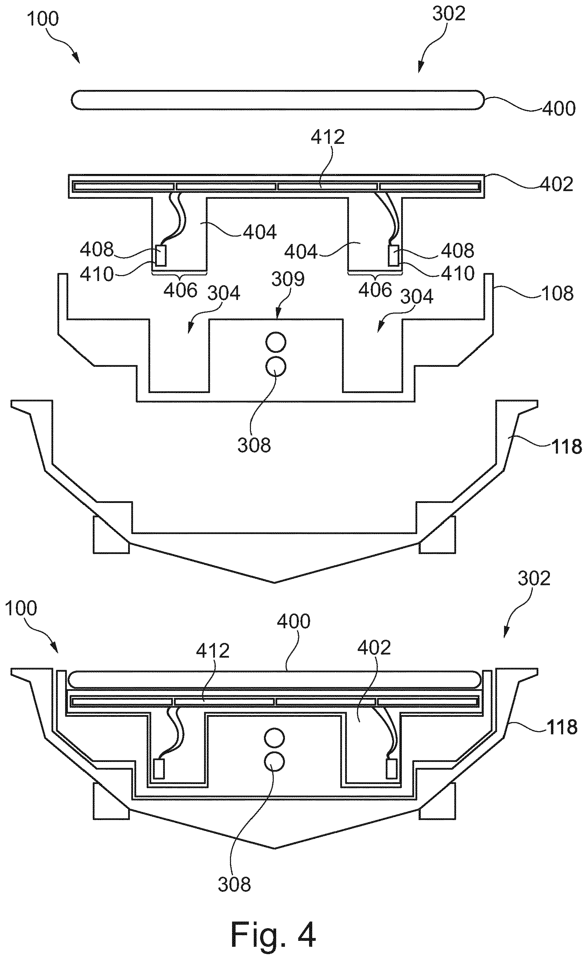

Fig. 4 shows a further cross-sectional view 302 of the subject support 100. The subject support 100 is shown in both an assembly and an exploded view. The bridge 118 which is part of the magnet and the table top 108 from Fig. 3 are shown again. However, in this example a modular table insert 402 has been inserted into the table top 108. An optional mattress 400 is shown as being on top of the modular table insert 402. The modular table insert 402 has two mechanical extensions 404 which fit into the grooves 304. The mechanical extensions 404 each have an end region 406. There is an electronic portion 408 and a table insert connector 410 at the end regions 406. Placing the electronics 408 in the end region 406 keeps any heat generating components further away from where the subject would repose on the table top 108. The connectors 410 would connect with one of the connectors 306 depicted in Fig. 3. The modular table inserts 402 are shown as comprising a magnetic resonance imaging coil 412.

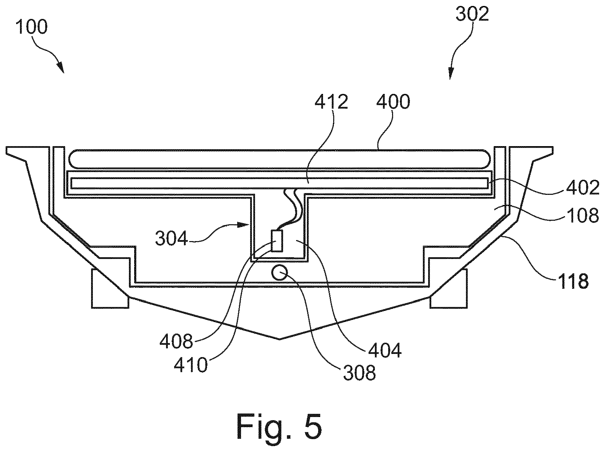

As mentioned above, Fig. 4 illustrates an exploded view of the cross-section 302 of the tabletop 108 and a generic coil tile (modular table insert 402). As shown, the protrusions (mechanical extensions 404) in the tile 402 are formed in a manner to avoid, where possible, being directly below the center line of a subject lying on the table. However, an alternative which shows this variation is illustrated in Fig. 5 below.

The protrusions may be formed as a pair running the length 110 of the coil tile 402 and towards each edge of the tile leaving a central gap. The central gap facilitates a convenient mechanical, electrical, optical and thermal interfacing to a complementary mechanical interface on the subject tabletop that extends along its length (the CMS 308). The tabletop interface contains a complementary central island (central support structure 309) and a pair of recesses (troughs 304) either side. The central island 309 of the tabletop provides complementary mechanical, electrical, optical and thermal interfaces for connecting with one or multiple tiles 402 placed along the length of the tabletop.

The dimensions of an individual tiles 402 are fixed/standardized with respect to the cross-section and interface to the tabletop 108. The length of a tile, however, can be variable depending on the requirements of the coil 412 it forms. A coil tile 402 can contain any number of elements and also structure and antennas 412 integrated above the surface e.g. a head coil.

The form factor of examples may make efficient use of the space below the subject and ensures that the bulk of coil electronics can be positioned as far away from the subject and imaging volume as possible. Coil electronics like pre-amps and digital receivers can generate heat during use and may keep these heat sources as far from the subject as possible and primarily not directly below the center line of the subject. By using the protrusion/trough approach it also becomes possible to provide a simple air cooling interface to remove heat from within the coils without requiring explicit connections above the tabletop or within the subject space. Auxiliary coil electronics can also contain ferromagnetic material. A further goal of this invention is to facilitate placing those components as far from the imaging volume as possible without taking space away from the subject bore.

Fig. 5 shows a further example of the subject support 100. In this example the table top 108 only has one groove 304 and the modular table insert 402 only has a single mechanical extension 404. For example, the cable system 308 may in some examples only provide a digital or fiber optic communication cable. The benefit of moving the cable system as close to the center line and as far away from any body coil is then removed. All of the designs shown with two mechanical extensions 404 could be modified as is illustrated in Fig. 5. Likewise, variants with two, three, four and five mechanical extensions 404 can also be created.

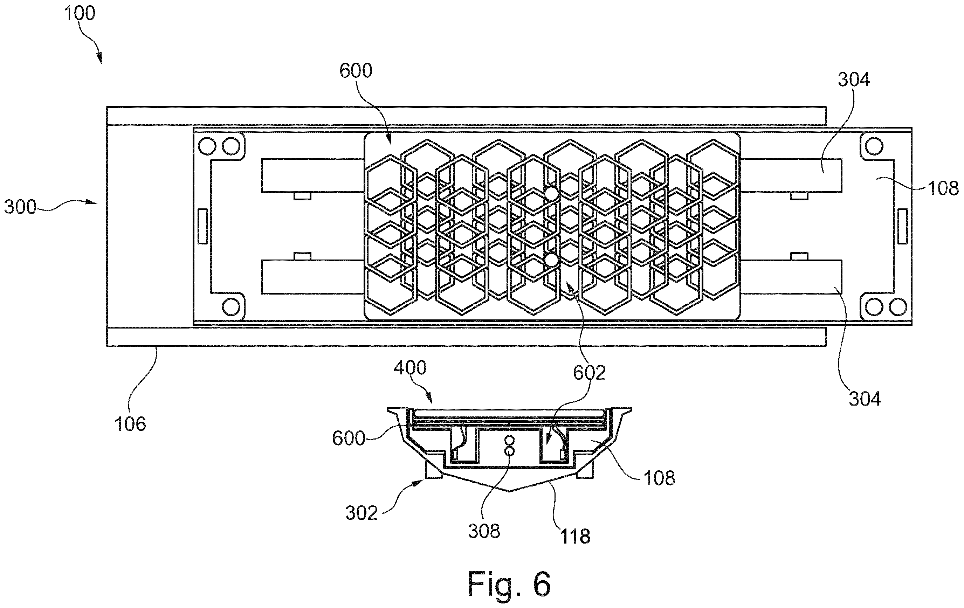

Fig. 6 illustrates a further top view 300 of the subject support 100. In this example a spine coil modular table insert 600 has been inserted into the table top 108. The spine coil modular table insert 600 provides a surface coil which may be used as a spine coil. The region 602 indicates the location of a ventilation recess 602 that travels within the grooves 304. This enables efficient cooling of the electronic portion of the spine coil modular table insert 600.

The spine coil 600 (spine coil modular table insert 600) of Fig. 6 extends half the length of the tabletop. An additional base coil tile (modular table insert) could extend one quarter the length of the tabletop.

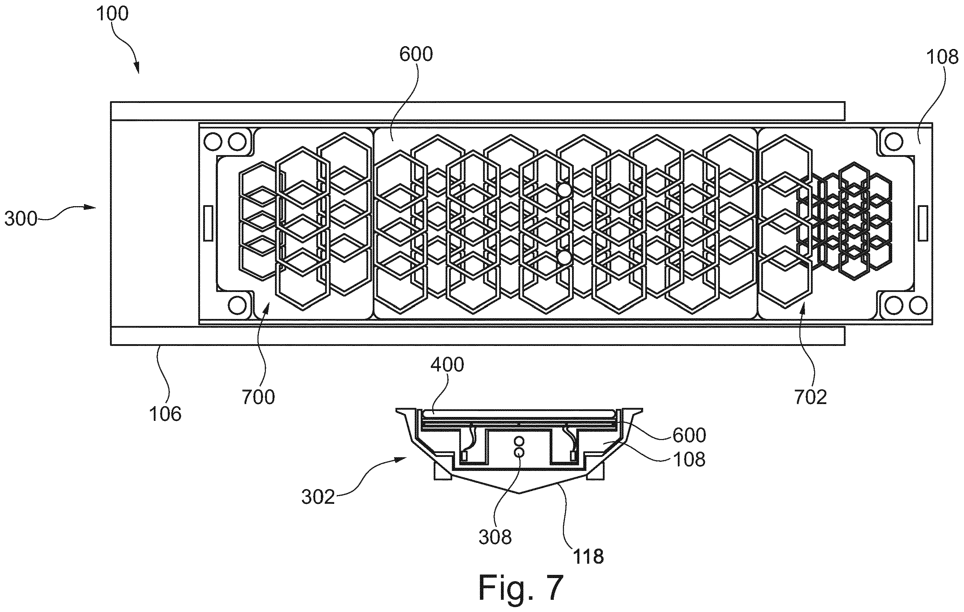

In Fig. 6 it can be seen that there are two portions of the table top 108 that are not covered with inserts. Fig. 7 illustrates the same subject support as shown in Fig. 6 but two additional inserts have been inserted into the table top 108. The table top 108 can further be seen as having a base coil modular table insert 700 that has been installed and a head-neck coil modular table insert 702 that has also been installed.

In Fig. 7, a head coil is integrated onto a base coil tile (head-neck coil modular table insert 702) and all connections take place below the surface thereby eliminating the need for cables and conventional connectors.

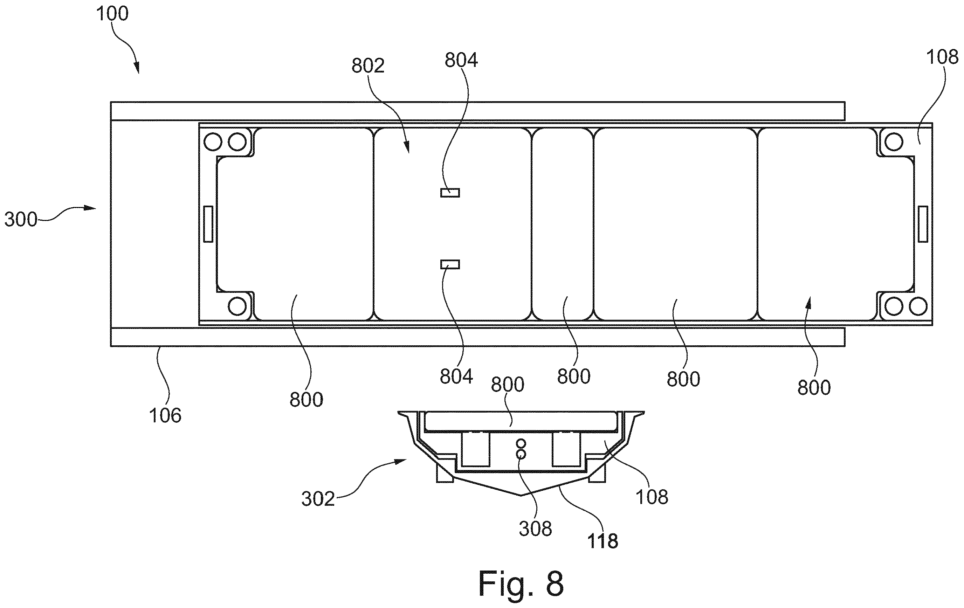

Fig. 8 illustrates some alternative inserts that can be used or placed in the table top 108. In this example there are a number of thick mattresses or mattress structures which take the place of a modular table insert. They essentially are spacers which fill the volume of the table top 108 and allow it to be used without any active electronics or coils. In this example there is a single knee coil connection insert 802 that either provides connectors 804 on its surface for connecting a knee coil or integrates the knee coil into the modular table insert 802. The rest of the table top 108 is filled with the thick mattresses or mattress structures 800. The connector 804 is connected to the table insert connector 410 which is not shown.

The knee or foot/ankle coil (a single knee coil connection insert 700) is integrated onto a base tile with supporting electronics located in the bottom protrusions 404 of the tile and interfacing to the central island 309 on the tabletop 108 without the need of cables. At locations where coils are not required, a replaceable filler or mattress 400 is used.

Coils that are placed on top of the subject, like the anterior coil, are connected to the subject carrier using a short cable and connector to sockets located at each of the four corners of the subject carrier using the Standard DCIs (see figure 3). Alternatively, an anterior coil may be battery powered and connected wirelessly to a receiver contained within a base coil tile.

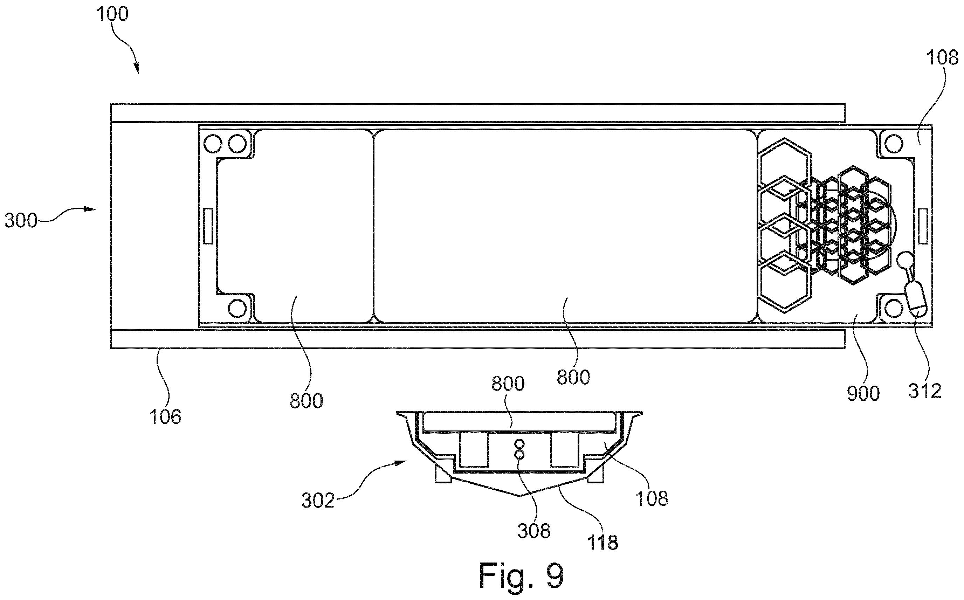

Fig. 9 shows an alternate configuration of the subject support 100. In this example at one of the ends a multinuclear head coil tile 900 has been inserted into the table top 108. The remaining space of the table top 108 is filled with the thick mattresses or mattress structures 800. The multinuclear head coil tile 900 is shown as being connected to the analogue radio-frequency connector 312.

In an example, each tile (modular table insert 402) contains the digital receiving, detune and control electronics for the specific coil such that each coil is self-contained. In such a manner it becomes possible to independently evolve the antenna and receiver technology inside each coil tile without affecting the form factor or interfaces to the system. Furthermore, it may become possible, using the same subject carrier, to make use of coil tiles operating at different receiving frequencies as required for multi-nuclear imaging. This for example may be achieved using the example illustrated in Fig. 9. Since both the antenna and receiver contained inside the tile will be tuned to the particular Multi-Nuclear (MN) frequency, the user simply places the MN coil tile in place of a proton tile. It can also be that the MN requires transmit (Tx) functionality. This is facilitated by incorporating a separate Tx cable into the tile which is connected to one or more of the Tx/Rx connectors 312 located on the tabletop 108.

The MN Tx/Rx head coil tile (multi nuclear head tile 900) depicted in Fig,. 9 may connect below the tile for the Rx (Receive) functionality and may connect to the Tx/Rx connector 312 on the tabletop for the Tx (transmit) functionality. Such a coil may be tuned to the desired MN frequency, the Rx interface to the tabletop will be identical to that of a proton coil. RF transmit functionality is provided via the tabletop Tx/Rx connector 312.

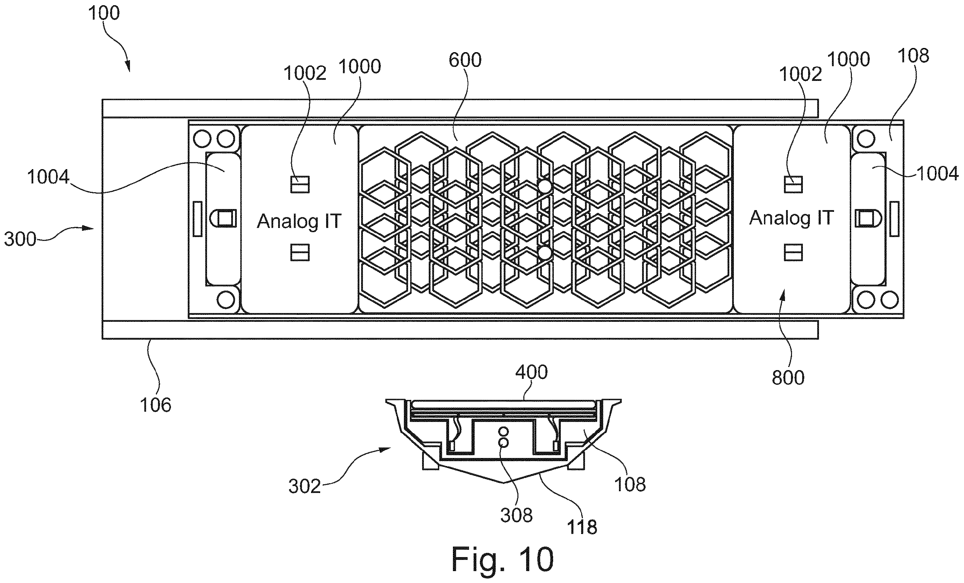

Fig. 10 illustrates a further configuration of the subject support 100. In this example the spinal coil modular table insert 600 is shown as being inserted again. On either side there is an analogue connection modular table insert 1000 which has analogue connections 1002 to either the ends of the table top 108 or to the cable system 308 if the cable system includes RF analogue connections. There are also some additional spacers 1004 to fill in the open space of the table top 108.

As illustrated in Fig. 10, a tile (modular table insert) can also form an analogue to digital interface by incorporating an analogue connector 1002 on its upper surface thereby allowing for the connection of analogue coils. Fig. 10 shows an illustration of one of many possible examples of such an analog interface tile (AIT) (analog connection modular table insert 1000). The generic nature of the tile form factor means that such an analogue interface tile 1000 could be placed at any position/station along the length of the subject carrier and multiple such tiles can be accommodated simultaneously.

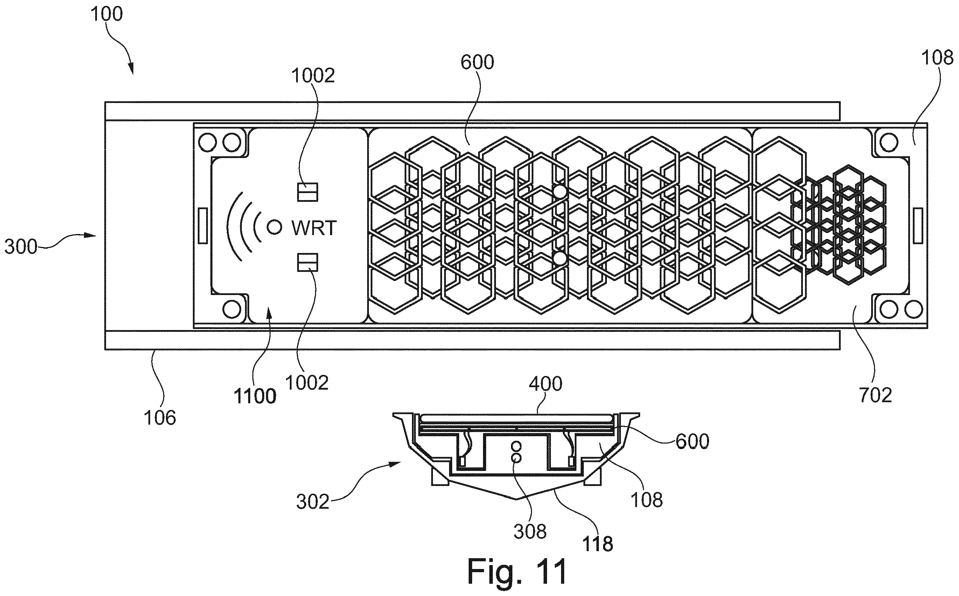

Fig. 11 illustrates an alternate configuration of the subject support 100. In this example the subject support 100 is similar to the configuration shown in Fig. 7 except the base coil modular table insert 700 has been replaced with a wireless modular table insert 1100. The wireless modular table insert 1100 connects to the cable system 308 and provides a wireless connection. This for example may be used for controlling magnetic resonance imaging coils that are wireless.

Any tile (any modular table insert) can also incorporate a wireless transceiver of Fig. 11 for wireless connection to a suitable coil placed on the subject thereby eliminating the need for cables and connectors. Since all frequency dependent processing and conversion to digital data takes place inside the coil tiles, the interface to the tabletop is both frequency and channel count agnostic and thereby greatly simplified. The Tx/Rx functionality provided on the tabletop may be broadband.

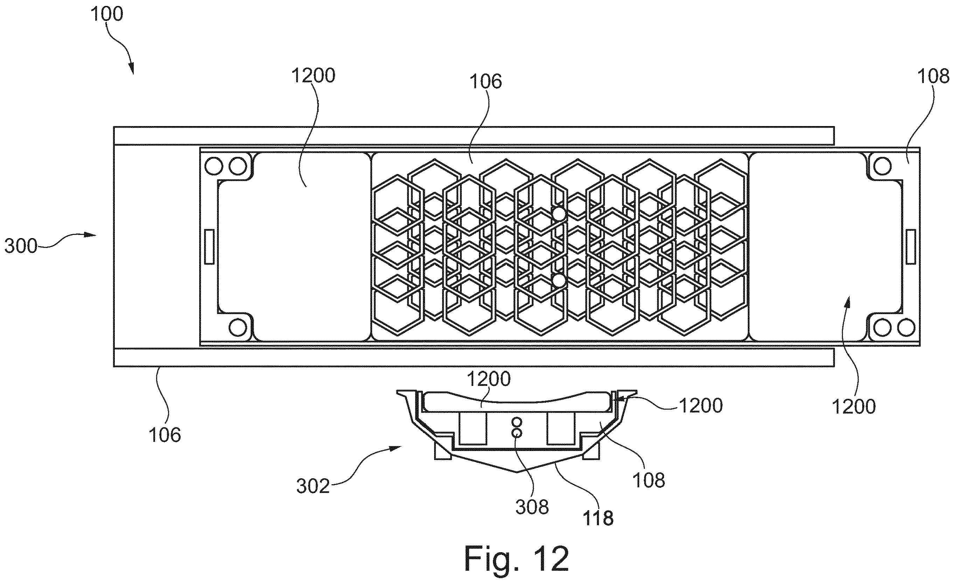

Fig. 12 illustrates a further configuration of the subject support 100. The configuration shown in Fig. 12 is similar to that of Fig. 6. In this example the empty spaces of the table top 108 in Fig. 6 have been filled with curved table spacers 1200. The curved table spacers 1200 are curved spacers which may be used for providing a particularly curved surface that may for example be useful for connecting with legacy magnetic resonance imaging coil or other equipment. The use of the curved table spacers 1200 for example allows complete customization of the surface of the table top 108.

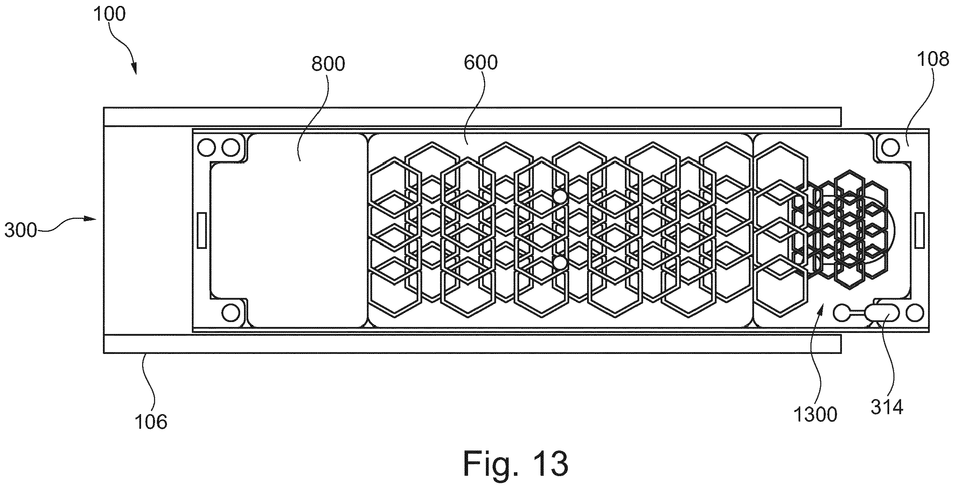

Fig. 13 illustrates a further configuration of the subject support 100. In this example the spine coil 600 and one thick mat 800 have been installed. At the other end a legacy coil modular table insert 1300 has been installed. This legacy coil modular table insert 1300 accepts a legacy coil and connects this coil via the connection 314.

Tabletop spacers 1200, 1300 as illustrated in Figs. 12 and 13 may accommodate legacy coils (magnetic resonance imaging coils). Such legacy coil may not have the requisite mechanical interface to the tabletop nor the Direct Connect DCI interface. In this case it is possible to utilize table spacers 1200, 1300 which serve to convert the mechanical interface of the legacy coil to match the flat tabletop. Fig.12 illustrates the use of tabletop spacers 1200, 1300 to accommodate legacy coils, and the Direct Connect DCI concept is briefly illustrated in Fig. 13.

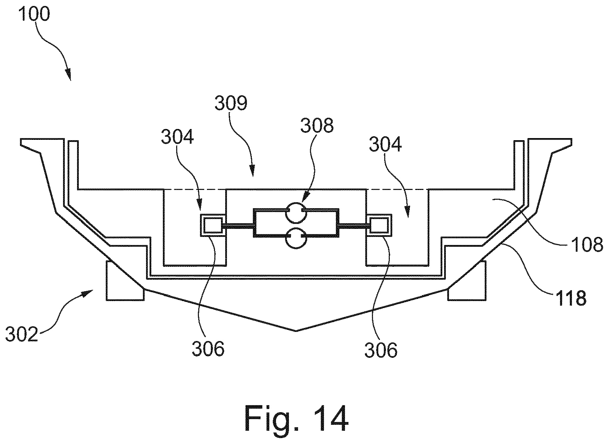

Fig. 14 illustrates one way of implementing the cable system 308. The cable system 308 is shown as travelling or passing through the central region of the central support structure 308. There are grooves 304 on either side. Connectors 306 are spaced periodically within the grooves 304. The connectors 306 may also be placed symmetrically within the grooves 304. This may enable the placement of modular table inserts in two orientations, placing them in one orientation and then rotating them on a 180°. This may enable greater flexibility in placing the modular table inserts.

The CMS (Cable Management System) (cable system 308) forms the backbone of the tabletop and consists of a central channel carrying power supply cables, data fibers, a galvanic cable power supply, possibly a digital signal transmitted on galvanic connection, possibly an ethernet cable such as a CAT7 ethernet cable, and, potentially, air cooling pipes. The CMS 308 may be located as close to the system iso-center line (iso-center line of the magnet) as possible to minimize interactions with the body coil. It is typically populated with RF traps to eliminate potentially hazardous interactions with the subject. The architecture facilitates the possibility to tap off power and data access at period points along its length. A suitably designed interface 306 protrudes into the trough space where it can connect with a complimentary interface 306 on each of the coil tiles 402, 600, 700, 702, 800, 900 1000. Such an interface can be galvanic or galvanic and optical.

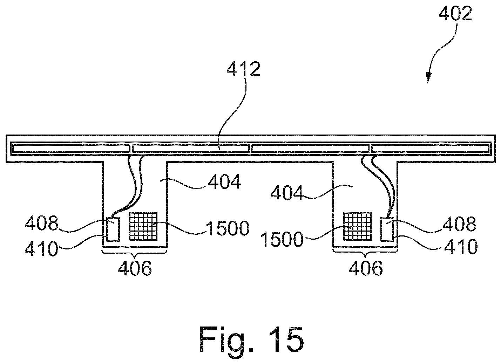

Fig. 15 illustrates a modification to the modular table insert 402 of Fig. 4. In this example in the end region 406 of the mechanical extensions 404 there are air vents 1500. The installation of the air vents 1500 may be beneficial because they may enable the passage of air through the grooves 304 even when the modular table inserts 402 have been installed. This may facilitate the cooling of the electronic portions 408.

The island portion 309 of the tabletop 108 can also contain air channels 602 with separate inlet and outlet interfaces which provide for air flow through the coil tiles. Alternatively, a negative air pressure can be established at one end of each trough 304 thereby forcing air flow through the protrusions 404 of each coil tile which would then contain ventilation holes (air vent 1500) at each end of the protrusion as is depicted in Fig. 15.

Examples may have one or more of the following benefits:
- The vast majority of coils are connected without the need for any cables at all. All connections take place in the troughs (groove) below the table top.
- The tabletop is fully digital with the exception of power and is frequency and channel count agnostic with respect to data. This makes it both simple, largely re-usable at any frequency and independent of coil tile technology (future proof).
- The tabletop troughs provide a robust standard mechanical/electrical interface and facilitate all system electro-optical and cooling interfaces below the table surface and out of sight.
- The concept makes the best use of the ample unused space below the tabletop.
- The coil tile with protrusions provides a larger volume for coil electronics and facilitates placing electronics like digital receivers as far away from the subject space as possible without taking additional space in the bore. This facilitates lower heat load and better immunity to ferromagnetic materials contained within some of the electronics.
- The dual protrusions/troughs design ensures that heat generating components are distributed towards the edge of the subject and not directly below the center line where the subject perception may be more sensitive. The trough design, in combination with ventilation holes in the coil tile protrusions facilitates a means to circulate air to better control the internal temperature of a coil tile and facilitate a larger number of channels than the static thermal load would otherwise allow.
- The coil tile concept facilitates many coil types which can be placed in a variety of combinations.
- The coil tile concept facilitates to build any type of interface to the system e.g. analog, wireless, physiological, etc. that can be exchanged at will and combined with other types of coil tile.
- Upgrade of coil technology is easily facilitated by replacing a tile containing old technology with one containing new technology.
- Coils that operate at different MR frequencies e.g. MN coils, can be integrated onto a dedicated tile with their appropriate digital receiver. Such a MN tile also integrates easily with the standard interface and can be used in combination with other coils.
- Tx/Rx connectors are integrated into the tabletop.
- provides air vents for cooling coil electronics
- The docking connector 120 may be simple, consisting of a single power connection, a single optical data connection and up to two RF coaxial cables supplying the Tx/Rx connector. Digital data could also be transmitted via the galvanic connection. Alternatively, the broadband RF power amplifier module could be built into the subject support.

Examples may incorporate one or more of the following features:
1. A feature of a combined subject support and modular coil platform incorporating a tabletop and configurable array of hollow coil tiles (modular table insert). Said coil tiles being replaceable/removable and consisting of a flat upper surface and undulating lower surface consisting of one or more protrusions (mechanical extensions 404) that form a complementary mechanical interface with respect to one or more troughs (grooves 304) located along the length of the tabletop.
2. A coil tile design for the platform of feature 1 wherein the upper surface of a coil tile incorporates coil antennas/elements (magnetic resonance imaging coil 412) and the lower protrusions contain auxiliary electronics (electronic portion 408) and a variety of power, data and cooling interfaces that provide for connection to the system, below the tabletop and without the need for cables.
3. A coil tile as in feature 2 which incorporates coil elements that extend above the surface of the tile.
4. The coil tile of feature 2 in which the auxiliary electronics consist of at least one digital receiver.
5. A platform as described in feature 1 which contains electrical, optical and thermal interfaces at periodic intervals within each of the troughs. Said interfaces designed to connect with complementary interfaces within the protrusions of a coil tile.
6. A platform as described in feature 1 which incorporates a central cable management system to serve the interfaces in the tabletop.
7. A platform as described in feature 1 which contains means (ventilation recess 602) to transport air along the troughs in the tabletop for the purpose of cooling electronics.
8. A coil tile as in feature 2 in which the protrusions incorporate ventilation holes (air vent 1500) for air cooling in combination with the platform of claim 7.
9. A coil tile (600) as in feature 2 for spine imaging.
10. A coil tile (700) as in feature 2 for lower leg imaging.
11. A coil tile (702) as in feature 2 for head-neck imaging.
12. A coil tile (802) as in feature 2 for knee imaging.
13. A coil tile (700) as in feature 2 for foot/ankle imaging.
14. A coil tile as in feature 2 for shoulder imaging.
15. A coil tile (1000) as in feature 1 that incorporates an analog connector (1002) on its upper surface providing for connection of an analog coil to the system.
16. A coil tile as in feature 2 that incorporates an analog connector (1002) on its upper surface providing for connection of an analog coil to the system.
17. A coil tile (1100) as in feature 1 that incorporates a wireless receiver internally providing for connection of a wireless coil to the system.
18. A coil tile as in feature 2 that incorporates a wireless receiver internally providing or connection of a wireless coil to the system.
19. A coil tile as in feature 2 that is a transmit/receive coil.
20. A coil tile (900) as in feature 2 that operates at a different frequency to proton.
21. A coil tile as in feature 20 that is a transmit/receive coil.
22. A platform (medical instrument) as in feature 1 that includes spacer tiles (1200, 1300) to accommodate legacy coils.
23. A platform as in feature 1 that incorporates subject biometric sensors.
24. A platform as in feature 1 incorporating a docking connector (120) consisting of a single power line, a single optical line and a single RF coaxial line.
25. A platform as in feature 1 including one or more mattresses (400) for covering the upper surface of the tiles.
26. A platform as in feature 1 including a series of empty/dummy tiles (800) used to fill the parts of the tabletop when a coil tile, or other functional tile, is not used.
27. A platform as in feature 1 including a series of thick mattresses (800) used to fill the parts of the tabletop when a coil tile, or other functional tile, is not used.

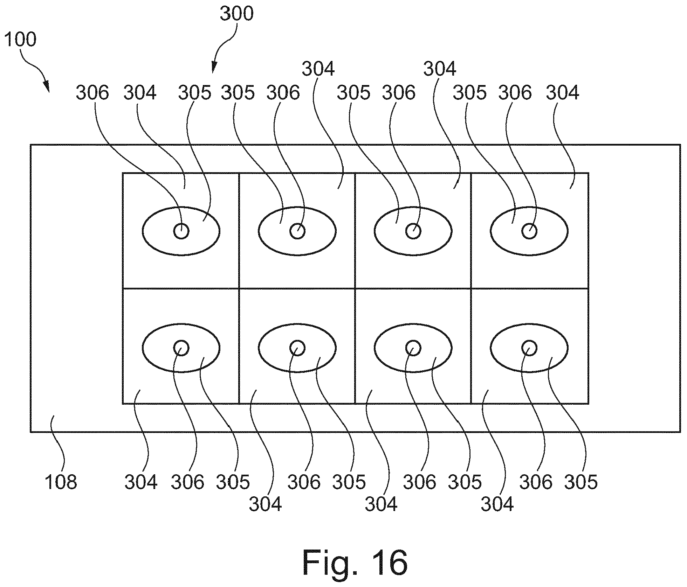

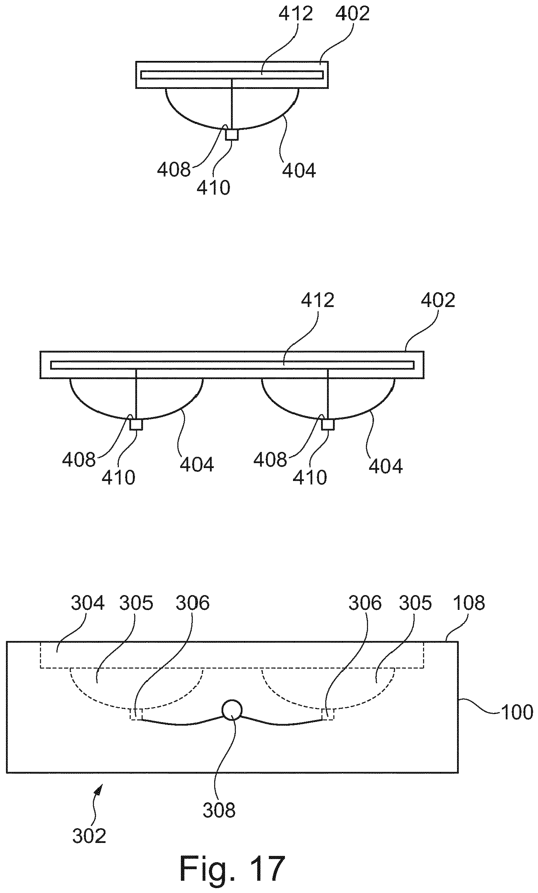

Figs. 16 and 17 show a top 300 and a cross-sectional view 302 of an alternative example of a subject support 100. In this example there are a number of receptacles 304 distributed along the subject support 100. The receptacles 304 each have apertures 305. At the bottom of each aperture 305 is a connector 306. The connector 306 connects to the cable system 308. It is not shown in this Fig. but each connector 306 could for example be protected or covered by a flap that is able to be pushed out of place by a connector on one of the modular table inserts 402. The apertures 305 can be shaped to mate or to join with modular table inserts 402. In Fig. 17 the modular table inserts 402 have mechanical extensions 404 that fit to match the apertures 305. There are two modular table inserts 402 illustrated in Fig. 17. The top one has a single mechanical extension 404 that is used to fill a single location or single receptacle 304. The bottom modular table insert 402 contains two mechanical extensions 404 and can be placed and connected filling two receptacles 304.

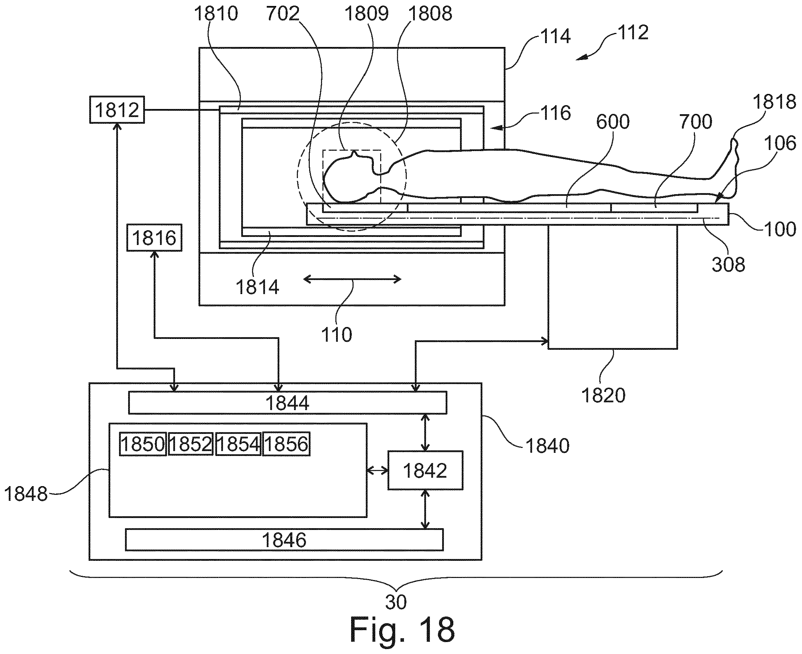

Fig. 18 shows a further example of a medical instrument 30. The medical instrument 30 comprises the magnetic resonance imaging system 112 and the subject support 100. In this example the magnetic resonance imaging system 112 comprises a magnet 114. The magnet 114 is a superconducting cylindrical type magnet with a bore 106 through it. The use of different types of magnets is also possible. For instance, it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 116 of the cylindrical magnet 114 there is an imaging zone 1808 where the magnetic field is strong and uniform enough to perform magnetic resonance imaging. A region of interest 1809 is shown within the imaging zone 1808. The magnetic resonance data that is acquired typically acquired for the region of interest. A subject 1818 is shown as being supported by a subject support 100 such that at least a portion of the subject 1818 is within the imaging zone 1808 and the region of interest 1809.

Within the bore 116 of the magnet there is also a set of magnetic field gradient coils 1810 which is used for acquisition of preliminary magnetic resonance data to spatially encode magnetic spins within the imaging zone 1808 of the magnet 504. The magnetic field gradient coils 510 connected to a magnetic field gradient coil power supply 1812. The magnetic field gradient coils 1810 are intended to be representative. Typically, magnetic field gradient coils 1810 contain three separate sets of coils for spatially encoding in three orthogonal spatial directions. A magnetic field gradient power supply supplies current to the magnetic field gradient coils. The current supplied to the magnetic field gradient coils 1810 is controlled as a function of time and may be ramped or pulsed.

The subject support 100 is further shown as comprising an actuator 820 which moves the subject support 100 into the bore of the magnet 116. The subject 1818 is then at least partially moved into the imaging zone 1808 of the magnetic resonance imaging system 112. The subject support 100 has a spine coil modular table insert 600, a base coil modular table insert 700, and a head-neck coil modular table insert 702 that has been inserted into the tabletop 106. The individual modular table inserts 600, 700, 702 each connect to the cable system 308. In this example the cable system 308 provides power and digital connectivity to each of the modular table inserts 600, 700, 702. The pickup coils in the subject support 100 can then be reconfigured on the fly by inserting different modular table inserts into them. The digital connectivity 308 of the cable enables the subject support 100 to be modified and scaled in terms of the number of channels on the fly as various modular table inserts are inserted or removed from the tabletop 106.

The bore or the magnet contains a body coil 1814 which may be a bird cage type body coil 1814 for manipulating the orientations of magnetic spins within the imaging zone 1808 and for receiving radio transmissions from spins also within the imaging zone 508. The body coil 1814 by be replaced by general purpose or specialized magnetic resonance imaging coils. The body coil 1814 may be optional and have its transmit functionality replaced by a modular table insert or other radio frequency coils connected to the subject support.

Instead of a body coil 1814 a different type radio frequency antenna may be used, for example a specialized coil that contains contain multiple coil elements for transmitting and/or receiving may be used. The radio frequency antenna may also be referred to as a channel or antenna. The body coil 1814 is connected to a radio frequency transceiver 1816. The body coil 1814 and radio frequency transceiver 1816 may be replaced by separate transmit and receive coils and a separate transmitter and receiver. It is understood that the body coil 1814 and the radio frequency transceiver 1816 are representative. The radio-frequency coil 1814 is intended to also represent a dedicated transmit antenna and a dedicated receive antenna. Likewise, the transceiver 1816 may also represent a separate transmitter and receivers. The body coil 1814 may also have multiple receive/transmit elements and the radio frequency transceiver 1816 may have multiple receive/transmit channels. For example, if a parallel imaging technique such as SENSE is performed, the radio-frequency coil 1814 will have multiple coil elements.

The medical instrument 30 is shown as further comprising a computer 1840. The computer 1840 comprises a processor 1842. The processor 1842 is intended to represent one or more processors or processing cores as well as processors which may be distributed amongst one or more computers. The processor 1842 is connected to a hardware interface 1844 which enables the processor to communicate with and control the various components of the magnetic resonance imaging system 112. The processor 1842 is also shown as being connected to an optional user interface 1846. The processor 1842 is further shown as being connected to a memory 1848. The memory 1848 may represent any combination of volatile or non-volatile memory which the processor 1842 can access. The memory 1840 is further shown as containing machine-executable instructions 1850. The machine-executable instructions contain program code and instructions that enable the processor 1842 to control the operation and function of the magnetic resonance imaging system. For example, the hardware interface 1844 is shown as being connected to the subject support 100, the magnetic field gradient coil power supply 1812 and the transceiver 1816. The processor 1842 can therefore control the acquisition of magnetic resonance imaging data using the hardware interface 1844. In particular the hardware interface 1844 forms a link with the cable system 308. This enables digital communication between the processor 1842 and each of the modular table inserts 600, 700, 702. This enables the on the fly scaling of the number of channels of the magnetic resonance imaging system 112.

The memory 1848 is further shown as containing pulse sequence commands 1852. The pulse sequence commands are instructions which enable the processor 1842 to control the magnetic resonance imaging system 112 to acquire magnetic resonance imaging data. The memory 1848 is further shown as containing magnetic resonance imaging data 1854 that has been acquired by controlling the magnetic resonance imaging system 112 with the pulse sequence commands 1852. The memory 1848 is further shown as containing a magnetic resonance image 1856 that has been reconstructed using the magnetic resonance imaging data 1854.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 10: medical instrument
- 20: medical instrument
- 30: medical instrument
- 100: subject support
- 102: transport
- 104: pedestal
- 106: carrier
- 108: tabletop
- 110: translational axis
- 112: magnetic resonance imaging system
- 114: magnet
- 116: bore of magnet
- 118: bridge
- 120: connector for cable system
- 300: top view
- 302: cross sectional view
- 304: receptacle or groove
- 305: aperture
- 306: connector
- 308: cable system (cable management system)
- 309: central support structure
- 310: opposing end region
- 312: Analog radio frequency connector
- 314: Connection to cable system
- 316: Nurse call/headphone connection
- 400: mattress
- 402: modular table insert (coil tile)
- 404: mechanical extension
- 406: end region
- 408: electronic portion
- 410: table insert connector
- 412: magnetic resonance imaging coil
- 600: spine coil modular table insert (tile)
- 602: ventilation recess
- 700: base coil modular table insert
- 702: head-neck coil modular table insert
- 800: thick mattress
- 802: knee coil connection insert
- 804: connector
- 900: multi nuclear head coil tile
- 1000: analog connection modular table insert
- 1002: analog connection
- 1004: spacer modular table insert
- 1100: wireless modular table insert
- 1200: curved table spacers
- 1300: legacy coil modular table insert
- 1500: air vent
- 1808: imaging zone
- 1809: region of interest
- 1810: magnetic field gradient coils
- 1812: magnetic field gradient coil power supply
- 1814: body coil
- 1816: transceiver
- 1818: subject
- 1820: actuator
- 1840: computer
- 1842: processor
- 1844: hardware interface
- 1846: user interface
- 1848: memory
- 1850: machine executable instructions
- 1852: pulse sequence commands
- 1854: magnetic resoance imaging data
- 1856: magnetic resonance image

## Claims

1. A medical instrument (10, 20, 30) comprising a subject support (100), wherein the subject support comprises a table top (108) configured for translating at least a portion of a subject within an imaging zone of a magnetic resonance imaging system (112),
wherein the table top comprises:
- as least one receptacle (304) recessed into the table top;
- a cable system (308) configured for providing power and/or connectivity to at least one magnetic resonance imaging coil (412); and
- multiple connectors (120) distributed within the at least one receptacle,
wherein the multiple connectors are configured for providing access to the power and/or the connectivity of the cable system.

2. The medical instrument of claim 1, wherein the cable system provides power and digital connectivity to the at least one magnetic resonance imaging coil.

3. The medical instrument of claim 2, wherein the digital connectivity is configured for providing the addition of magnetic resonance imaging channels to the medical instrument on the fly.

4. The medical instrument of claim 1, 2, or 3, wherein the medical instrument further comprises a magnetic resonance imaging system (112) configured for acquiring magnetic resonance imaging data from the imaging zone, wherein the magnetic resonance imaging system is configured to send and receive magnetic resonance imaging coil control commands via the connectivity of the cable system.

5. The medical instrument of any one of the preceding claims, wherein the table top is configured for translating the table top along a translational axis, wherein the at least one receptacle is at least one groove, wherein the at least one groove is parallel or perpendicular to the translational axis, multiple connectors (120) distributed parallel or perpendicular to the translational axis within each of the at least one groove.

6. The medical instrument of claim 5, wherein the as least one groove recessed into the table top is two grooves recessed into the table top, wherein the cable system is positioned within a central support structure (309) of the table top that runs parallel to the translational axis, wherein the central support structure is formed between the two grooves, wherein the two grooves are parallel to the translational axis.

7. The medical instrument of any one of claims 1 through 4, wherein the at least one receptacle is multiple apertures, wherein one of the multiple connectors is distributed in each of the multiple apertures.

8. The medical instrument of claim 1, 2, or 3, further comprising at least one modular table insert (402, 700, 802, 900, 1000, 1004, 1100, 1200, 1300) configured for being inserted into a receptacle of the at least one receptacle of the table top.

9. The medial instrument of any one of the preceding claims, wherein the at least one receptacle is configured for receiving the at least one module table insert using any one of the following: a gravitationally secured fit, a snap fit, and combinations thereof.

10. The medical instrument of any one of the preceding claims, wherein each of the at least one groove is configured (602, 1500) to provide air cooling.

11. The medical instrument of any of the preceding claims, wherein the table top is dockable to the magnetic resonance imaging system.

12. The medical instrument of any one of the preceding claims, wherein the table top comprises two opposing end regions (310), wherein at least one of the two opposing end regions comprises any one of the following: a connection to the cable system (314), an analog radio frequency connector (312), and combinations thereof.

13. A modular table insert (402, 700, 802, 900, 1000, 1004, 1100, 1200, 1300) configured for being inserted into at least one receptacle of the table top (108) of a medical instrument (10, 20) according to any of the previous claims, wherein the at least one receptacle is configured for receiving the least one modular table insert, wherein the at least one module table insert is configured for forming a support surface configured for receiving the subject, and wherein each of the at least one modular table insert is configured for connecting to one of the multiple connectors, wherein the modular table insert comprises a mechanical extension (404) for least one receptacle of the table top, wherein each mechanical extension is configured for being inserted into one of the at least one receptacle of the table top, wherein the modular table insert comprises a table insert connector (410) configured for connecting to one of the multiple connectors when inserted into the table top.

14. The modular table insert of claim 13, wherein the modular table insert comprises a magnetic resonance imaging coil (412) connected to the table insert connector.

15. The modular table insert of claim 14, wherein the magnetic resonance imaging coil is any one of the following: a surface coil, a head coil (702), a neck coil (702), a foot coil (700), a knee coil (8002), and a spine coil (600).

16. The modular table insert of any one of claims 13, 14, or 15, wherein the modular table insert further comprises any one of the following: an analog magnetic resonance imaging coil connector (1002), a WI-FI connector (1100), a digital magnetic resonance imaging coil connector (804), and combinations thereof.

17. The modular table insert of any one of claims 13 through 16, wherein the modular table insert further comprises an electronic portion (408), wherein the electronic portion is located in an end region (406) of the mechanical extension.

18. The modular table insert of any one of claims 13 through 17, wherein the modular table insert further comprises any one of the following: a contoured table surface (1200) and a mounting structure (1300) for a surface mountable magnetic resonance imaging coil.
